# DEMANDE DE BREVET EUROPEEN

(11) **EP 2 264 171 A1**
(43) Date de publication de la demande: **22.12.2010**
(21) Numéro de dépôt: 10172940.8
(22) Date de dépôt: 12.12.2006
(51) Int. Cl.: C12N 15/11, C07H 21/00, A61K 31/7105, A61K 8/60, A61P 17/00, A61Q 19/02

(54) **siRNA anti Myosine Va et depigmentation de la peau**

(30) Priorité: 12.12.2005 FR 0512553
(62) Demande divisionnaire de: 06830566.3
(71) Demandeur: Pierre Fabre Dermo-Cosmétique, 92100 Boulogne-Billancourt (FR)
(72) Inventeur: SCHMITT-MILAS, Anne-Marie, 31770, TOURNEFEUILLE (FR); LAMBERT, Jo, B-9840, DE PINTE (BE); WESTBROEK, Wendy, ROCKVILLE, MD 20853 (US); VAN GELE, Mireille, 9991, ADEGEM (Maldegem) (BE)
(74) Mandataire: Ahner, Francis

(57) **Abrégé**

La présente invention concerne de nouveaux siRNA isolés comprenant un brin d'ARN sens et un brin d'ARN antisens complémentaire formant ensemble un duplex d'ARN, **caractérisé en ce que** le brin d'ARN sens comprend une séquence qui possède au plus un nucléotide distinct par rapport à un fragment de 14 à 30 nucléotides contigus de la séquence nucléotidique de l'exon F du gène codant pour la protéine myosine Va, ainsi que des compositions comprenant au moins un tel siRNA, et l'utilisation d'au moins un tel siRNA comme agent cosmétique ou thérapeutique pour la dépigmentation de la peau.

LISTE DE SEQUENCES

<110> PIERRE FABRE DERMO-COSMETIQUE
      SCHMITT-MILAS, Anne-Marie
      LAMBERT, Jo
      WESTBROEK, Wendy
      VAN GELE, Mireille

<120> SiRNA anti myosine Va et dépigmentation de la peau

<130> 350579-D23968

<150> FR 0512553
<151> 2005-12-12

<160> 20

<170> PatentIn version 3.3


<210> 1
<211> 75
<212> ADN
<213> homo sapiens

<220>
<223> Séquence de l'exon F de la protéine myosine Va humaine

<400> 1
tattttgagg aattatatgc agatgaccct aagaagtatc aatcatatcg gatttccctt      60
tacaaacgga tgatt                                                       75


<210> 2
<211> 19
<212> ADN
<213> homo sapiens

<220>
<223> fragment de l'exon F de la protéine myosine Va humaine

<400> 2
tgaccctaag aagtatcaa                                                   19


<210> 3
<211> 19
<212> ADN
<213> homo sapiens

<220>
<223> fragment de l'exon F de la protéine myosine Va humaine

<400> 3
gtatcaatca tatcggatt                                                   19


<210> 4
<211> 19
<212> ADN
<213> homo sapiens

<220>
<223> fragment de l'exon F de la protéine myosine Va humaine

<400> 4
tcatatcgga tttcccttt                                                    19


<210> 5
<211> 19
<212> ADN
<213> séquence artificielle

<220>
<223> amorce sens d'amplification de l'exon F de la protéine
      myosine Va humaine

<400> 5
cagcctgcag cacgagatc                                                    19


<210> 6
<211> 27
<212> ADN
<213> séquence artificielle

<220>
<223> amorce antisens d'amplification de l'exon F de la
      protéine myosine Va humaine

<400> 6
tcttagggtc atctgcatat aattcct                                           27


<210> 7
<211> 23
<212> ADN
<213> séquence artificielle

<220>
<223> amorce sens d'amplification de la partie GP de la protéine
      myosine Va humaine

<400> 7
gcagtcaatt tgattccagg att                                               23


<210> 8
<211> 25
<212> ADN
<213> séquence artificielle

<220>
<223> amorce antisens d'amplification de la partie GP de la
      protéine myosine Va humaine

<400> 8
tgatcatcat tcaggtagtc agcat                                             25


<210> 9

<211> 21
<212> ARN
<213> séquence artificielle

<220>
<223> brin sens siRNA anti-myosine Va n°1

<400> 9
ugacccuaag aaguaucaat t                                                21


<210> 10
<211> 21
<212> ARN
<213> séquence artificielle

<220>
<223> brin antisens siRNA anti-myosine Va n°1

<400> 10
uugauacuuc uuagggucat t                                                21


<210> 11
<211> 21
<212> ARN
<213> séquence artificielle

<220>
<223> brin sens siRNA anti-myosine Va n°2

<400> 11
guaucaauca uaucggauut t                                                21


<210> 12
<211> 21
<212> ARN
<213> séquence artificielle

<220>
<223> brin antisens siRNA anti-myosine Va n°2

<400> 12
aauccgauau gauugauact t                                                21


<210> 13
<211> 21
<212> ARN
<213> séquence artificielle

<220>
<223> brin sens siRNA anti-myosine Va n°3

<400> 13
ucauaucgga uuucccuuut t                                                21


<210> 14
<211> 21

<212> ARN
<213> séquence artificielle

<220>
<223> brin antisens siRNA anti-myosine Va n°3

<400> 14
aaagggaaau ccgauaugat t                                                21


<210> 15
<211> 56
<212> ADN
<213> séquence artificielle

<220>
<223> brin sens oligonucléotide pour clonage dans un vecteur
      lentiviral d'un shRNA anti-myosine Va

<400> 15
gatcgtatca atcatatcgg attctcaaga gaaatccgat atgattgata cttttt          56


<210> 16
<211> 56
<212> ADN
<213> séquence artificielle

<220>
<223> brin antisens oligonucléotide pour clonage dans un vecteur
      lentiviral d'un shRNA anti-myosine Va

<400> 16
agctaaaaag tatcaatcat atcggatttc tcttgagaat ccgatatgat tgatac          56


<210> 17
<211> 47
<212> ARN
<213> séquence artificielle

<220>
<223> shRNA anti-myosine Va

<400> 17
guaucaauca uaucggauuc ucaagagaaa uccgauauga uugauac                    47


<210> 18
<211> 55
<212> ADN
<213> séquence artificielle

<220>
<223> brin sens oligonucléotide pour clonage dans un vecteur
      lentiviral d'un shRNA contrôle brouillé

<400> 18
gatcattatc taggagatat cacctcaaga gagtgatatc tcctagataa ttttt           55

<210> 19
<211> 56
<212> ADN
<213> séquence artificielle

<220>
<223> brin antisens oligonucléotide pour clonage dans un vecteur
      lentiviral d'un shRNA contrôle brouillé

<400> 19
agctaaaaaa ttatctagga gatatcactc tcttgaggtg atatctccta gataat          56


<210> 20
<211> 46
<212> RNA
<213> séquence artificielle

<220>
<223> shRNA contrôle brouillé

<400> 20
auuaucuagg agauaucacc ucaagagagu gauaucuccu agauaa                     46

## Description

La présente invention concerne de nouveaux siRNA isolés comprenant un brin d'ARN sens et un brin d'ARN antisens complémentaire formant ensemble un duplex d'ARN, **caractérisé en ce que** le brin d'ARN sens comprend une séquence qui possède au plus un nucléotide distinct par rapport à un fragment de 14 à 30 nucléotides contigus de la séquence nucléotidique de l'exon F du gène codant pour la protéine myosine Va, ainsi que des compositions comprenant au moins un tel siRNA, et l'utilisation d'au moins un tel siRNA comme agent cosmétique ou thérapeutique pour la dépigmentation de la peau.

La pigmentation de la peau est un processus complexe comprenant de nombreuses étapes. Les mélanocytes, qui sont situés dans la couche basale de l'épiderme, peuvent être activés par des stimuli internes ou externes (par exemple, une exposition aux ultraviolets (UV)) à produire de la mélanine dans des organelles spécialisés apparentés aux lysosomes appelés les mélanosomes. Après maturation, les mélanosomes périnucléaires sont transportés le long des microtubules et du cytosquelette d'actine jusqu'à la périphérie des dendrites des mélanocytes (Lambert J et al. Cell Mol Biol (Noisy-le-grand): 45: 905-18, 1999; Hara M et al. J Invest Dermatol.: 114:438-43, 2000; Vancoillie G et al. J Invest Dermatol.: 114: 421-9, 2000). Les mélanosomes, qui contiennent la mélanine, sont ensuite transférés aux kératinocytes adjacents depuis les extrémités des dendrites par un mécanisme encore inconnu.

Toute altération d'une étape de ce processus complexe peut aboutir à des troubles de la pigmentation de la peau. En particulier, une augmentation du nombre de mélanocytes actifs, et/ou une augmentation de la production de mélanine, et/ou une augmentation du transfert de mélanosomes des mélanocytes vers les kératinocytes peuvent conduire à une hyperpigmentation (ou dyschromatose) de la peau. Une telle hyperpigmentation peut être induite par de nombreux facteurs, tels que les médicaments, le soleil, des disfonctionnements métaboliques ou nutritionnels, ou encore par des maladies génétiques ou auto-immunes. Cela implique généralement des conséquences cosmétiques et psychologiques importantes pour le patient, qui va dans de nombreux cas chercher un traitement adéquat.

De nombreux efforts ont donc été faits pour mettre au point des traitements efficaces contre l'hyperpigmentation. La plupart des traitements chimiques existant comprennent des agents dépigmentants ciblant la voie de synthèse de la mélanine, notamment en inhibant l'activité de l'enzyme tyrosinase qui est nécessaire à la synthèse de la mélanine. De tels agents dépigmentants incluent notamment l'hydroquinone et ses dérivés, le rétinol ou la trétinoine, l'acide ascorbique et ses dérivés, des extraits placentaires, l'acide kojique, l'acide férulique, l'arbutine, des dérivés de dihydroxybenzène (WO 00/47045), des dérivés du guaiacol (WO 00/47179), le 4-(2, 3-dihydroxyphényl)-cyclohexanol (WO 00/56279), des dérivés du résorcinol (WO 00/56702), des amides phénoliques (WO 99/32077). Ces substances peuvent présenter certains inconvénients. Elles peuvent être instables, nécessiter une utilisation à des concentrations élevées, manquer de spécificité quant à leur mode d'action, ou présenter un pouvoir cytotoxique ou irritant.

Des traitements de type physique ont également été mis au point, tels que l'exfoliation de profondeur moyenne, la dermabrasion ou la thérapie laser. Cependant, ces traitements sont généralement moins efficaces et peuvent causer des effets secondaires gênants tels que des risques d'hypo- ou d'hyperpigmentation post-inflammatoires, une ochronose, ou une cicatrisation pouvant provoquer des effets carcinogènes (Briganti S et al. Pigment Cell Res.: 16: 101-10, 2003; Halder RM, Nootheti PK. J Am Acad Dermatol.: 48: S143-48, 2003; Halder RM, Richards GM. Skin Therapy Lett.: 9:1-3, 2004).

Il existe donc un besoin de mise au point de nouveaux traitements cosmétiques et/ou thérapeutiques de l'hyperpigmentation qui soient efficaces et sûrs.

Outre les traitements chimiques, une autre possibilité pour obtenir l'inhibition de l'expression d'un gène donné consiste à utiliser des acides nucléiques antisens ou à tirer profit d'un processus appelé ARN interférence (Dykxhoorn DM et al. Nat Rev Mol Cell Biol.: 4: 457-67, 2003; Soutschek J et al. Nature: 432: 173-8, 2004).

L'ARN interférence (appelée dans toute la suite RNAi) est le processus par lequel un ARN double brin (dsRNA) de séquence nucléique sens donnée induit la dégradation de tous les ARN messager (ARNm) comprenant ladite séquence nucléique, d'une manière spécifique de ladite séquence nucléique. Bien que le processus de RNAi ait initialement été mis en évidence chez *Caenorhabditis elegans*, il est maintenant clair que le processus de RNAi est un phénomène très général, et l'inhibition de l'expression de gènes humains par RNAi a été obtenue.

Le processus de RNAi peut être obtenu en utilisant de petits ARN interférants (ou siRNA). Ces siRNA sont des dsRNA de moins de 30 nucléotides comprenant dans leur séquence sens une séquence fortement homologue, de préférence identique, à un fragment de l'ARNm ciblé. Lorsqu'un siRNA traverse la membrane plasmique, la réaction de la cellule est de détruire le siRNA ainsi que toutes les séquences comportant une séquence identique ou fortement homologue. Ainsi, un ARNm possédant un fragment identique ou fortement homologue à la séquence du siRNA sera détruit, l'expression de ce gène étant ainsi inhibée.

Pour le traitement de l'hyperpigmentation, il a été proposé dans la technique antérieure d'utiliser des oligonucléotides antisens dirigés contre les gènes de la tyrosinase ou de la protéine TRP-1 (WO 01/58918), ou contre le gène de la protéine PKC β1 qui est impliquée dans le processus de phosphorylation de la tyrosinase (WO 2005/073243). Il a également été proposé d'utiliser des siRNA ciblant le gène de la tyrosinase (WO 2005/060536).

Cependant, tous ces gènes sont impliqués dans la voie de synthèse de la mélanine, et il n'a jamais été divulgué ni suggéré de siRNA ciblant un gène impliqué, non pas dans la voie de synthèse de la mélanine, mais dans le transport des mélanosomes au sein des mélanocytes et leur transfert vers les kératinocytes.

Il a été montré, notamment par les inventeurs, que la protéine myosine Va est impliquée dans le transport des mélanosomes au sein des mélanocytes (Lambert J et al. Cell Mol Biol (Noisy-le-grand): 45: 905-18, 1999; Lambert et al. J Invest Dermatol.: 111: 835-40, 1998). Plus précisément, la protéine myosine Va possède plusieurs variants d'épissage, et le variant comportant l'exon F est nécessaire au transport des mélanosomes (Westbroek W et al. J Jnvest Dermatol.: 120: 465-75, 2003).

Jusqu'à présent cependant, aucune tentative n'avait été faite pour inhiber l'expression du variant de la protéine myosine Va comportant l'exon F en utilisant des siRNA, ni pour appliquer cette approche au traitement cosmétique ou thérapeutique de l'hyperpigmentation.

Les inventeurs ont trouvé qu'il était possible de synthétiser des siRNA ciblant l'exon F de la protéine myosine Va et d'induire grâce à ces siRNA un processus de RNAi spécifique de l'exon F de la protéine myosine Va. Ils ont également montré que lesdits siRNA sont efficaces pour inhiber l'expression du variant de la protéine myosine Va comportant l'exon F à des concentrations de siRNA n'induisant pas de baisse de la viabilité cellulaire et que l'effet est dose-dépendant. De plus, les inventeurs ont également trouvé que la présence de siRNA ciblant l'exon F de la protéine myosine Va permet d'inhiber le transfert des mélanosomes des mélanocytes vers les kératinocytes.

L'invention concerne donc un siRNA isolé comprenant un brin d'ARN sens et un brin d'ARN antisens complémentaire formant ensemble un duplex d'ARN, **caractérisé en ce que** le brin d'ARN sens comprend une séquence qui possède au plus un nucléotide distinct par rapport à un fragment de 14 à 30, avantageusement 15 à 29, 16 à 28, 17 à 27, 18 à 25, 18 à 23, ou 18 à 21 nucléotides contigus de la séquence nucléotidique de l'exon F du gène codant pour la protéine myosine Va. Par « comprend un fragment de la séquence nucléotidique de l'exon F du gène codant pour la protéine myosine Va », on entend dans toute la description de l'invention le fait de comprendre une séquence d'ARN correspondant à la séquence génique, c'est-à-dire une séquence d'ARN qui correspond à la séquence génique dans laquelle les T sont remplacés par des U.

Le brin sens d'un siRNA selon l'invention comprend donc soit un fragment de 14 à 30, avantageusement 15 à 29, 16 à 28, 17 à 27, 18 à 25, 18 à 23, ou 18 à 21 nucléotides contigus de la séquence nucléotidique de l'exon F du gène codant pour la protéine myosine Va, soit une séquence présentant un nucléotide distinct par rapport à un tel fragment. En effet, le processus de RNAi est spécifique de la séquence, et une forte homologie de séquence est nécessaire pour obtenir une RNAi efficace. De manière avantageuse, le brin d'ARN sens comprend une séquence identique à un fragment de 14 à 30, avantageusement 15 à 29, 16 à 28, 17 à 27, ou 18 à 25 nucléotides contigus de la séquence nucléotidique de l'exon F du gène codant pour la protéine myosine Va.

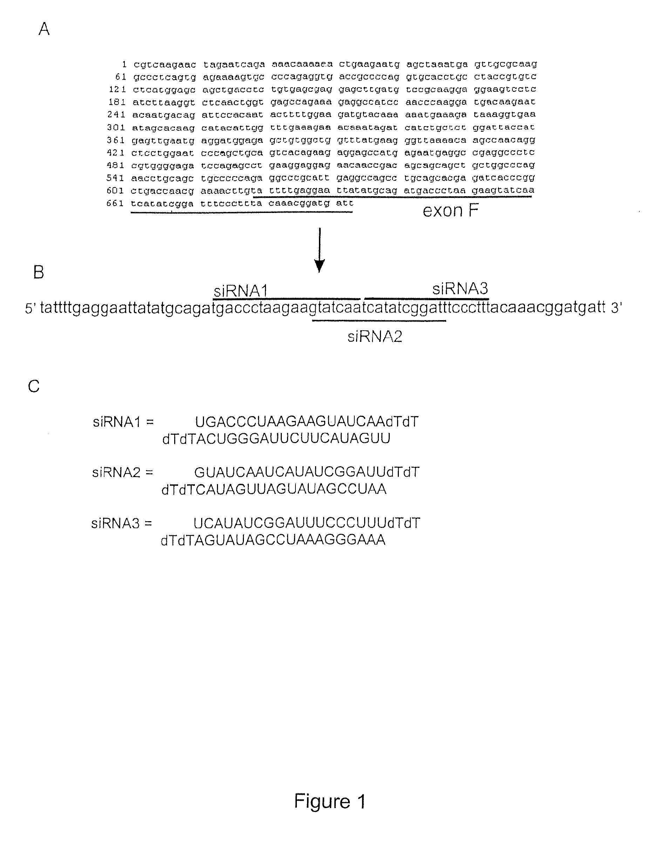

La protéine myosine Va a été décrite chez différentes espèces, en particulier l'Homme, la souris, le rat. Dans un mode de réalisation avantageux, la séquence nucléotidique de l'exon F du gène codant pour la protéine myosine Va est la séquence humaine de l'exon F de la protéine myosine Va, qui est représentée par SEQ ID N°1. La séquence de la protéine myosine Va humaine est représentée sur la Figure 1A, la portion correspondant à l'exon F (SEQ ID N°1) étant soulignée.

Tous les siRNA ciblant un fragment de 14 à 30, avantageusement 15 à 29, 16 à 28, 17 à 27, 18 à 25, 18 à 23, ou 18 à 21 nucléotides contigus de la séquence SEQ ID N°1 sont inclus dans la portée de l'invention. Toutefois, il est avantageux que le fragment de l'exon F de la protéine myosine Va qui est ciblé, c'est-à-dire dont la séquence est comprise dans le brin sens du siRNA, ne soit pas un fragment qui possède une structure caractéristique dans l'ARNm de la protéine myosine Va ou auquel viennent se lier des protéines régulatrices. Par « structure caractéristique », on entend au sens de l'invention une structure de type tige ou boucle ou épingle à cheveu capable de se former dans un ARNm de la protéine myosine Va. Différents outils sont mis à disposition gratuitement sur internet par plusieurs organisations (voir Tableau 1 ci-dessous), et peuvent être utilisés pour dessiner des siRNA ciblant l'exon F de la protéine myosine Va humaine.

**Tableau 1. sites internet mettant à disposition des outils de sélection de siRNA**

| Organisation | Adresse internet |
|---|---|
| Ambion | http://www.ambion.com/techlib/misc/siRNA_design.html |
| Dharmacon | http://www.dharmacon.com/sidesign/ |
| Qiagen | http://wwwl.qiagen.com/Products/GeneSilencing/CustomSiRna/ SiRnaDesigner.aspx |
| Emboss | http://bioweb.pasteur.fr/seqanal/interfaces/sirna.html |
| Tuschl Laboratory | http://www.rockefeller.edu/labheads/tuschl/sirna.html |
| The Whitehead | http://jura.wi.mit.edu/bioc/siRNAext/ |

La plupart des sociétés commercialisant des siRNA à façon garantissent l'efficacité de siRNA synthétisés. De plus, l'efficacité de tout siRNA ciblant l'exon F de la protéine myosine Va peut être vérifiée par différents essais, notamment ceux décrits en détail dans les exemples 1 et 2.

Les inventeurs ont synthétisé et testé plusieurs siRNA particuliers ciblant différents fragments de l'exon F de la protéine myosine Va humaine. Dans un mode de réalisation avantageux d'un siRNA selon l'invention, le fragment de 14 à 30, avantageusement 15 à 29, 16 à 28, 17 à 27, 18 à 25, 18 à 23, ou 18 à 21 nucléotides contigus de la séquence nucléotidique de l'exon F du gène codant pour la protéine myosine Va humaine est constitué d'une séquence nucléotidique choisie parmi SEQ ID N°2, SEQ ID N°3, et SEQ ID N°4. La position des trois séquences SEQ ID N°2, SEQ ID N°3, et SEQ ID N°4 est précisée sur la Figure 1B. Dans un mode de réalisation particulièrement avantageux, le fragment de 14 à 30, avantageusement 15 à 29, 16 à 28, 17 à 27, 18 à 25, 18 à 23, ou 18 à 21 nucléotides contigus de la séquence nucléotidique de l'exon F du gène codant pour la protéine myosine Va est constitué de la séquence nucléotidique SEQ ID N°3.

Un siRNA selon l'invention est un petit ARN double brin dont les brins sens et antisens sont appariés par des liaisons de type Watson-Crick, et dont la séquence du brin sens est constituée de ou comprend un fragment de 14 à 30, avantageusement 15 à 29, 16 à 28, 17 à 27, 18 à 25, 18 à 23, ou 18 à 21 nucléotides contigus de la séquence nucléotidique de l'exon F de la protéine myosine Va.

Il est connu que les siRNA dont la séquence est composée de 30 à 50 % de guanines et cytosines sont plus efficaces que les séquences avec une proportion plus importante de guanines et cytosines. Les siRNA selon l'invention possèdent donc avantageusement une séquence composée de 30 à 50 % de guanines et cytosines.

Il doit être compris qu'un siRNA selon l'invention peut indifféremment comprendre deux molécules d'ARN simple brin complémentaires, ou bien une seule molécule d'ARN simple brin dans laquelle deux portions complémentaires sont appariées par des liaisons de type Watson-Crick et sont liées d'un côté de façon covalente par une structure de type épingle à cheveux (on parle alors plus précisément de shRNA (pour « short hairpin RNA »), qui peut être considéré comme une sous-catégorie de siRNA). Dans toute la description, on entendra dons le terme siRNA dans son sens large incluant les shRNA, sauf indication contraire. Dans un mode de réalisation avantageux, un siRNA selon l'invention comprend deux molécules d'ARN simple brin complémentaires. Dans un autre mode de réalisation avantageux, un siRNA selon l'invention comprend ou est constitué d'une seule molécule d'ARN simple brin dans laquelle deux portions complémentaires sont appariées par des liaisons de type Watson-Crick et sont liées d'un côté de façon covalente par une structure de type épingle à cheveux, c'est-à-dire est un shRNA.

De plus, les brins d'ARN sens et/ou antisens peuvent comprendre en outre un fragment 3' en saillie de 2 à 4 nucléotides, en particulier lorsque un siRNA selon l'invention comprend deux molécules d'ARN simple brin complémentaires. Par « fragment 3' en saillie de 2 à 4 nucléotides », on entend la présence dans au moins un brin du duplex d'ARN de 2 à 4 nucléotides non appariés au brin complémentaire à l'extrémité 3' dudit brin. Les nucléotides utilisés dans le fragment 3' en saillie peuvent être des nucléotides naturels (ribonucléotides ou déoxyribonucléotides), ou des nucléotides modifiés tels que les LNA (Locked Nucleic Acid) qui comportent un pont méthylène entre les positions 2' et 4' du ribose (Soutschek J. et al. Nature. 2004 Nov 11;432(7014):173-8). Le fragment 3' en saillie peut également avoir subi tout type de modification chimique décrite dans le paragraphe suivant pour le brin d'ARN sens et/ou le brin d'ARN antisens d'un siRNA selon l'invention. Avantageusement, le fragment 3' en saillie est constitué de 2 nucléotides. Dans ce cas, des séquences préférées pour le fragment 3' en saillie sont « TT » (où T représente la déoxythymidine) ou « UU » (où U représente l'uracile). Avantageusement également, les deux brins complémentaires d'un siRNA selon l'invention comprennent un fragment 3' en saillie. Dans ce cas, la longueur et la séquence des deux fragments 3' en saillie peuvent être identiques ou différentes. De manière avantageuse, les deux brins complémentaires d'un siRNA selon l'invention comprennent chacun le même fragment 3' en saillie de 2 nucléotides de séquence « TT ». De tels siRNA comprenant les séquences ARN correspondant à SEQ ID N°2, SEQ ID N°3, ou SEQ ID N°4 et sur chaque brin un fragment 3' en saillie de séquence « TT » sont représentés sur la Figure 1C et correspondent à des modes de réalisation avantageux de l'invention. Ils correspondent :
- au siRNA MyoVa n°1 comprenant la séquence ARN de la séquence génique SEQ ID N°2 dont les brins ont pour séquences précises :
   brin sens : 5'-UGACCCUAAGAAGUAUCAATT-3' (SEQ ID N°9)
   brin antisens : 5'-UUGAUACUUCUUAGGGUCATT-3' (SEQ ID N°10);
- au siRNA MyoVa n°2 comprenant la séquence ARN de la séquence génique SEQ ID N°3 dont les brins ont pour séquences précises :
   brin sens : 5'-GUAUCAAUCAUAUCGGAUUTT-3' (SEQ ID N°11)
   brin antisens : 5'-AAUCCGAUAUGAUUGAUACTT-3' (SEQ ID N°12);
- au siRNA MyoVa n°3 comprenant la séquence ARN de la séquence génique SEQ ID N°4 dont les brins ont pour séquences :
   brin sens : 5'-UCAUAUCGGAUUUCCCUUUTT-3' (SEQ ID N°13)
   brin antisens : 5'-AAAGGGAAAUCCGAUAUGATT-3' (SEQ ID N°14);

Lorsque un siRNA selon l'invention est un shRNA constitué d'une seule molécule d'ARN simple brin dans laquelle deux portions complémentaires sont appariées par des liaisons de type Watson-Crick et sont liées d'un côté de façon covalente par une structure de type épingle à cheveux, c'est-à-dire lorsque le siRNA selon l'invention est un shRNA, ledit shRNA comprend ou est constitué de préférence de la séquence suivante :
5'-**GUAUCAAUCAUAUCGGAUU**CUCAAGAGA**AAUCCGAUAUGAUUGAUAC**-3' (SEQ ID N°17), où les portions en gras correspondent aux deux portions complémentaires appariées par des liaisons de type Watson-Crick, correspondant à la ARN de séquence génique SEQ ID N°3 comprise dans le brin sens du siRNA MyoVa n°2 et sa séquence complémentaire, et la portion non en gras correspond à la séquence de la structure de type épingle à cheveux liant les deux brins complémentaires.

En outre, dans un siRNA selon l'invention, le brin d'ARN sens et/ou le brin d'ARN antisens peuvent également comprendre au moins une modification chimique au niveau de leurs parties sucres, de leurs parties nucléobases ou de leur squelette internucléotidique. De telles modifications peuvent notamment permettre d'inhiber la dégradation des siRNA par les nucléases in vivo. Toutes les modifications chimiques permettant d'améliorer la stabilité et la biodisponibilité in vivo des siRNA selon l'invention sont ainsi incluses dans la portée de l'invention. Parmi les modifications avantageuses dans les parties sucres, on peut notamment citer les modifications intervenant en position 2' du ribose, telles que les modifications 2'-deoxy, 2'-fluoro, 2'-amino, 2'-thio, ou 2'-O-alkyl, en particulier 2'-O-méthyle, au lieu du groupement 2'-OH normal sur les ribonucléotides, ou encore la présence d'un pont méthylène entre les positions 2' et 4' du ribose (LNA). Concernant les nucléobases, il est possible d'utiliser des bases modifiées, telles que notamment la 5-bromo-uridine, la 5-iodo-uridine, la N³-méthyl-uridine, une 2,6-diaminopurine (DAP), la 5-méthyl-2'-deoxyCytidine, la 5-(1-propynyl)-2'-deoxy-Uridine (pdU), la 5-(1-propynyl)-2'-deoxyCytidine (pdC), ou des bases conjuguées avec du cholestérol. Enfin, des modifications avantageuses du squelette internucléotidique comprennent le remplacement de groupements phosphodiesters de ce squelette par des groupements phosphorothioate, méthylphosphonate, phosphorodiamidate, ou l'utilisation d'un squelette composé d'unités de N-(2-aminoethyl)-glycine liées par des liaisons peptidiques (PNA, Peptide Nucleic Acid). Les différentes modifications (base, sucre, squelette) peuvent bien entendu être combinées pour donner des acides nucléiques modifiés de type morpholino (bases fixées sur un cycle morpholine et liées par des groupes phosphorodiamidate) ou PNA (bases fixées sur des unités de N-(2-aminoethyl)-glycine liées par des liaisons peptidiques).

Les siRNA selon l'invention sont « isolés », ce qui signifie qu'ils ne sont pas à un état naturel mais ont été obtenus par tout moyen impliquant une intervention humaine. Notamment, les siRNA selon l'invention peuvent avoir été obtenus par purification de siRNA préexistants, par synthèse chimique, par amplification des séquences nucléotidiques désirées par une réaction de polymérisation en chaîne (PCR), ou par synthèse recombinante. De nombreuses sociétés proposent également la synthèse de siRNA à façon, telles que notamment les sociétés Eurogentec, Ambion, Dharmacon, ou Qiagen.

L'invention concerne également les siRNA selon l'invention, en tant qu'agent cosmétique. Par « agent cosmétique », on entend une substance permettant d'entretenir, ou d'améliorer l'aspect esthétique d'une partie externe du corps humain ou animal.

L'invention concerne en outre les siRNA selon l'invention, en tant que médicament. En effet, les siRNA selon l'invention sont nouveaux en tant que médicament.

L'invention a aussi pour objet une composition comprenant au moins un siRNA selon l'invention et un support acceptable. Par « support acceptable », on entend tout support cosmétologiquement ou pharmacologiquement acceptable connu de l'homme du métier.

Une composition selon l'invention est destinée au traitement cosmétique et/ou thérapeutique de la peau et est donc avantageusement administrée par voie topique.

Une composition pour application topique selon l'invention peut être formulée sous toute forme galénique habituellement utilisée pour une application topique, comme par exemple sous forme d'une solution aqueuse, d'une crème blanche ou colorée, d'une pommade, d'un lait, d'une lotion, d'un gel, d'un onguent, d'un sérum, d'une pâte, d'une émulsion huile dans l'eau ou eau dans l'huile, ou d'une mousse. Elle peut éventuellement être appliquée sur la peau sous forme d'aérosol. Elle peut également se présenter sous forme solide pulvérulent ou non, par exemple sous forme de stick. Elle peut encore se présenter sous la forme de patchs, de crayons, de pinceaux et d'applicateurs autorisant une application localisée sur les taches du visage ou des mains.

Une composition selon l'invention peut en outre comprendre tout type de véhicule connu de l'homme du métier permettant d'améliorer la délivrance et la biodisponibilité des siRNA selon l'invention. Des véhicules particuliers pouvant être utilisés avec des siRNA comprennent notamment les liposomes et les peptides capables de franchir la membrane cellulaire (appelés CPP pour « Cell-permeable peptides »). Par « liposome », on entend une vésicule lipidique artificielle dont la membrane est constituée d'une ou plusieurs bicouches lipidiques et qui possède la capacité d'encapsuler et de protéger des protéines ou des acides nucléiques et de fusionner avec la membrane cellulaire, permettant ainsi de délivrer le produit encapsulé à l'intérieur des cellules. L'utilisation de liposomes permet donc de protéger les siRNA et de faciliter leur pénétration au sein des cellules. Par CPP on entend des peptides capables d'être internalisés et d'atteindre ensuite les compartiments cytoplasmique et/ou nucléaire de cellules vivantes. Des exemples de tels CPP comprennent les peptides Pénétratine, Transportan, Tat, MAP et SynB1. Des exemples particulièrement utiles de CPP consistent en des peptides amphipathiques dérivés de virus qui interagissent directement avec les acides nucléiques à délivrer pour former des nanoparticules capables de diffuser à travers la membrane plasmique. La conjugaison des siRNA selon l'invention à un quelconque CPP peut donc également permettre de protéger les siRNA et de faciliter leur entrée dans les cellules in vivo.

Une composition selon l'invention peut également comprendre un ou plusieurs actifs visant à renforcer les effets recherchés. Dans un mode de réalisation particulier, une composition selon l'invention peut notamment comprendre en outre au moins un autre agent dépigmentant. Par « agent dépigmentant », on entend toute substance qui agit directement sur les mélanocytes en inhibant soit leur activité, soit une des étapes de la voie de synthèse de la mélanine, soit encore le transport des mélanosomes vers les dendrites et leur transfert vers les kératinocytes. Parmi les agents dépigmentants connus susceptibles d'être ajoutés à une composition selon l'invention, on peut notamment citer l'hydroquinone et ses dérivés, le rétinol ou la trétinoine, l'acide ascorbique et ses dérivés, des extraits placentaires, l'acide kojique, l'acide férulique, l'arbutine, des dérivés de dihydroxybenzène (WO 00/47045), des dérivés du guaiacol (WO00/47179), le 4-(2, 3-dihydroxyphényl)-cyclohexanol (WO 00/56279), des dérivés du résorcinol (WO 00/56702), des amides phénoliques (WO 99/32077). D'autres agents dépigmentants susceptibles d'être ajoutés à une composition selon l'invention comprennent des oligonucléotides antisens ou des siRNA dirigés contre d'autres gènes que celui codant pour la protéine myosine Va impliqués dans une étape de la voie de synthèse de la mélanine ou dans le transport des mélanosomes vers les dendrites et leur transfert vers les kératinocytes, tels que notamment des oligonucléotides antisens ou des siRNA ciblant la tyrosinase (voir WO 01/58918 et WO 2005/060536), la protéine TRP-1 (voir WO 01/58918) ou la protéine PKC β1 (voir WO 2005/073243).

Les compositions selon l'invention peuvent également contenir d'autres substances actives ou excipients utilisés habituellement dans des compositions topiques destinées au soin de la peau telles que par exemple des filtres solaires chimiques ou physiques (tels que par exemple le méthoxycinnamate d'octyle, le butyl-méthoxydibenzoyl-méthane, l'oxyde de titane et l'oxyde de zinc), des agents antiglycation et/ou antioxydants pris seuls ou en association (tels que par exemple le tocophérol et ses dérivés, l'ergothionéine, la thiotaurine, l'hypotaurine, l'aminoguanidine, la thiamine pyrophosphate, la pyridoxamine, la lysine, l'histidine, l'arginine, la phénylalanine, la pyridoxine, l'adénosine triphosphate), des agents anti-inflammatoires (tels que par exemple le stéarylglycyrrhétinate), des agents apaisants et leurs mélanges, des agents conservateurs (anti-bactériens ou anti-fongiques), des agents hydratant, des modificateurs de pH, des agents kératolytiques et/ou desquamants (tels que par exemple l'acide salicylique et ses dérivé), des vitamines, des épaississants, des agents émollients, des eaux thermales, des tensioactifs, des polymères, des huiles de silicone, des huiles végétales, des huiles essentielles, des parfums, des matières colorantes ou des pigments, etc.

L'invention concerne également l'utilisation d'au moins un siRNA ou d'une composition selon l'invention comme agent cosmétique pour la dépigmentation ou le blanchiment de la peau. Les siRNA et les compositions selon l'invention peuvent être utilisés comme agent cosmétique pour la dépigmentation ou le blanchiment de la peau, soit de façon globale pour diminuer la pigmentation de la peau dans son ensemble, soit au niveau de zones hyperpigmentées, quelle que soit l'origine de l'hyperpigmentation desdites zones. Une telle utilisation permet en effet d'homogénéiser la pigmentation de la peau, améliorant ainsi son aspect esthétique. De plus, même en absence de zones hyperpigmentées, certaines personnes peuvent désirer une pigmentation globale plus claire, ce qui peut être obtenu par une utilisation cosmétique selon l'invention.

L'invention concerne en outre l'utilisation d'au moins un siRNA ou d'une composition selon l'invention pour la fabrication d'un médicament destiné au traitement ou à la prévention de l'hyperpigmentation de la peau, en particulier des hyperpigmentations épidermiques, des mélasmas et des hyperpigmentations post-inflammatoires. L'hyperpigmentation peut avoir pour origine de nombreux troubles différents. On peut notamment citer les mélanoses du visage et du cou, tels que le chloasma, la mélanose de Riehl, la Poikilodermie Reticulée Pigmentaire du Visage et du Cou, ou la Poïkilodermie héréditaire acrokératosique ; les éphélides ; les tâches café-au-lait ; les naevus de Sutton ; les hyperpigmentations dues à des causes métaboliques telles que l'hémochromatose, la maladie d'Addison, le syndrome de Cushing ou l'hyperthyréose ; et les hyperpigmentations dues à des causes exogènes telles que les hyperpigmentations d'origine médicamenteuse (substances telles que la chlorpromazine, les phénotiazines, l'hydantoïne, l'arsenic inorganique, les antimalariaux) ou par métaux lourds (argent, or, mercure); les hyperpigmentations post-inflammatoires induites après traumatismes, éruptions eczémateuses, lichen simple chronique, lupus érythémateux, et dermatoses incluant pytyriasis rosea, psoriasis, dermatite herpétiforme, érythème pigmenté fixe, et photo-dermatite ; le syndrome de Rothmund-Thomson ; l'acanthosis nigricans bénigne ou l'acanthosis nigricans maligne. L'utilisation d'un siRNA ou d'une composition selon l'invention pour la fabrication d'un médicament permet de traiter ces différentes pathologies et de faire disparaître les zones d'hyperpigmentation induites par ces troubles. Elle permet également de prévenir l'apparition de zones d'hyperpigmentation induites par ces différents troubles.

L'invention concerne également une méthode de traitement cosmétique destinée à la dépigmentation et au blanchiment de la peau, comprenant l'administration par voie topique d'une composition selon l'invention.

L'invention concerne en outre une méthode de traitement thérapeutique des hyperpigmentations de la peau, comprenant l'administration par voie topique d'une composition selon l'invention.

Les exemples suivants illustrent la présente invention sans toutefois la limiter.

### DESCRIPTION DES FIGURES

**Figure 1****.** Dessin de 3 siRNA ciblant l'exon F de la protéine myosine Va humaine. **A.** Séquence codante nucléotidique des transcrits de la protéine myosine Va humaine comprenant l'exon F (comprend les exons ABCDEF). La partie correspondant à l'exon F est soulignée. **B.** Détail de la séquence de l'exon F. Les positions correspondant aux trois siRNA choisis sont soit soulignées (siRNA MyoVa n°2 (SEQ ID N°3)) soit surlignées (siRNA MyoVa n°1 (SEQ ID N°2), ou MyoVa n°3 (SEQ ID N°4)). C. Structure des 3 siRNA choisis ciblant l'exon F de la protéine myosine Va humaine.

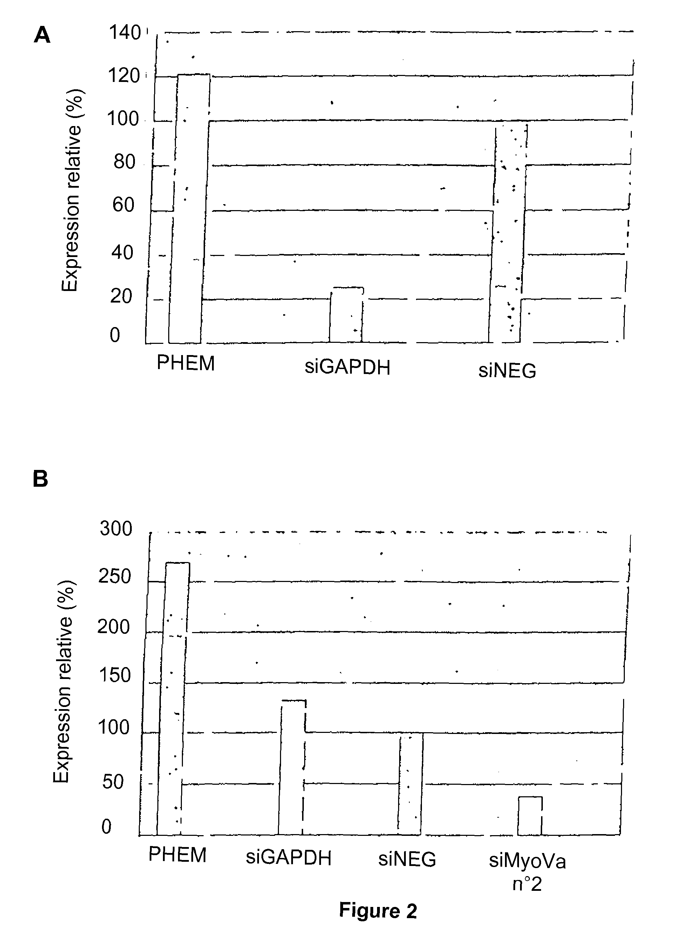

**Figure 2****.** Réduction de la quantité de transcrits de la protéine myosine Va comportant l'exon F dans des cellules MEHP électroporées avec 2 µM de siRNA MyoVa n°2 (SEQ ID N°3). Des cellules MEHP ont été électroporées avec 2 µM de siRNA contrôle négatif, de siRNA ciblant le gène GAPDH ou de siRNA MyoVa n°2 (SEQ ID N°3). L'effet des différents siRNA est mesuré au niveau ARN par QPCR. **A.** Niveau d'expression relatif du gène GAPDH en fonction du siRNA électroporé. **B.** Niveau d'expression des transcrits de la protéine myosine Va comportant l'exon F en fonction du siRNA électroporé.

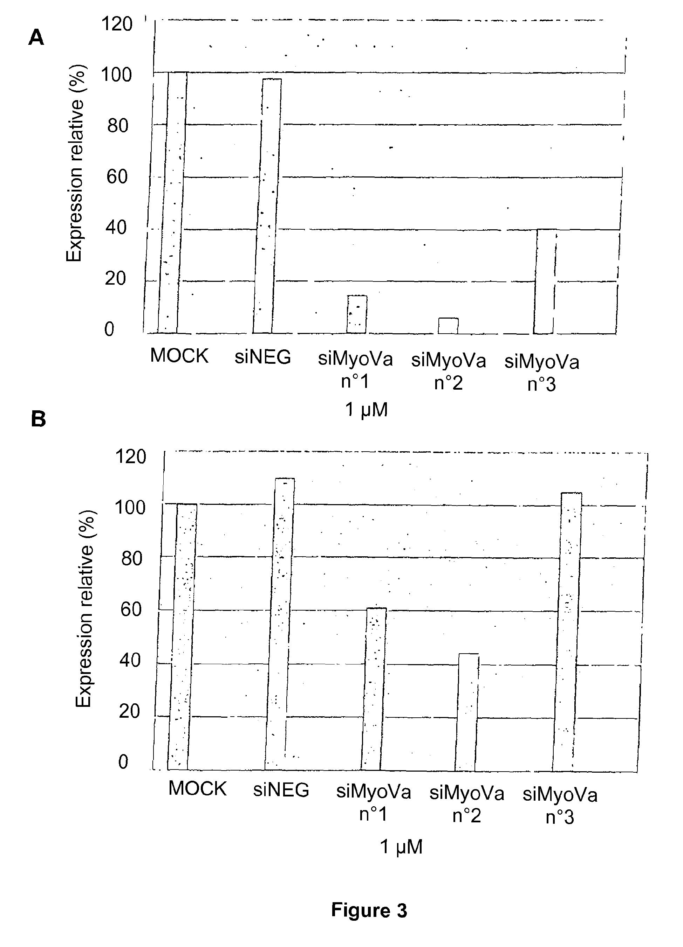

**Figure 3****.** Réduction spécifique de la quantité de transcrits de la protéine myosine Va comportant l'exon F, mais pas des transcrits ne comportant pas l'exon F. **A.** Niveau d'expression relatif des transcrits de la protéine myosine Va comportant l'exon F en fonction du siRNA électroporé. **B.** Niveau d'expression relatif de tous les transcrits de la protéine myosine Va par détection de la partie globulaire (GP) en fonction du siRNA électroporé.

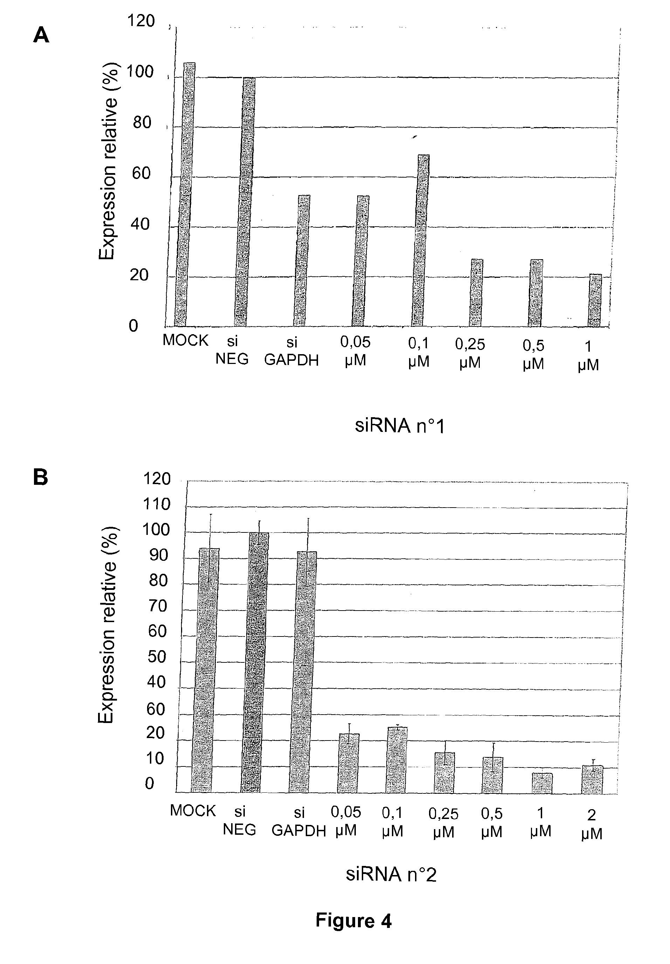

**Figure 4****.** Détermination de la concentration minimale ayant une efficacité maximale pour les siRNA MyoVa n°1 (SEQ ID N°2) et MyoVa n°2 (SEQ ID N°3). Différentes concentration de siRNA ciblant l'exon F de la protéine myosine Va ont été testées pour les siRNA **A.** MyoVa n°1 (SEQ ID N°2) et **B.** MyoVa n°2 (SEQ ID N°3). Pour MyoVa n°2, les résultats sont représentés sous forme de moyennes avec erreur standard.

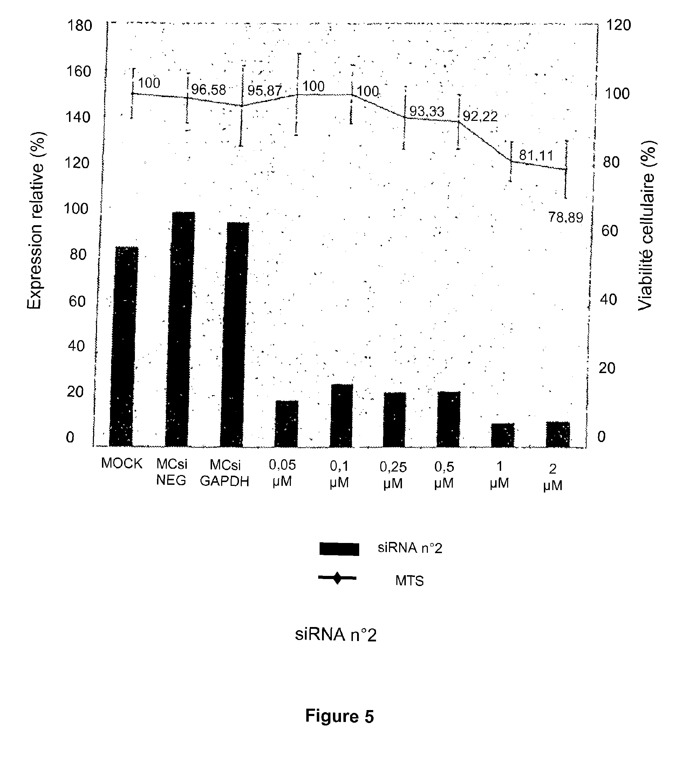

**Figure 5****.** Effet de la concentration en siRNA sur la viabilité des cellules. Le niveau d'expression relatif des transcrits comportant l'exon F et la viabilité des cellules ont été déterminés en parallèle pour différentes concentrations du siRNA MyoVa n°2 (SEQ ID N°3). Les concentrations en siRNA sont indiquées en abscisse, le niveau d'expression relatif des transcrits comportant l'exon F et la viabilité des cellules en ordonnées, respectivement sous forme d'un histogramme (barres grises) et d'une courbe représentant la valeur moyenne et l'erreur standard.

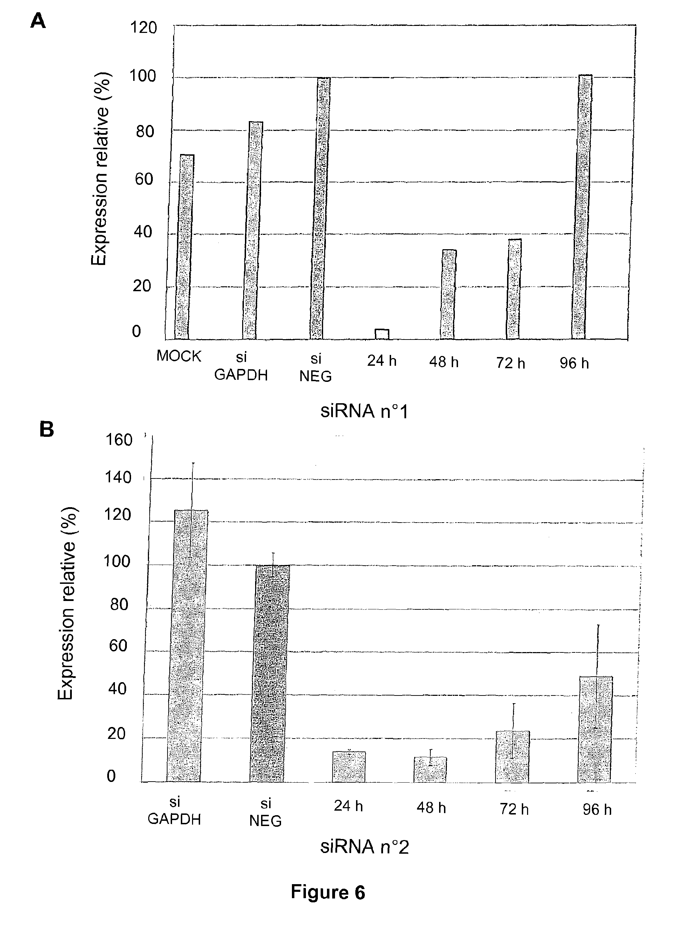

**Figure 6****.** Efficacité des siRNA ciblant l'exon F de la protéine myosine Va au cours du temps. Les cellules MEHP ont été analysées à différents temps après électroporation de 0,5 µM de siRNA MyoVa n°1 (SEQ ID N°2) ou MyoVa n°2 (SEQ ID N°3). **A.** Niveau d'expression relatif pour MyoVa n°1 (SEQ ID N°2). **B.** Niveau d'expression relatif pour MyoVa n°2 (SEQ ID N°3).

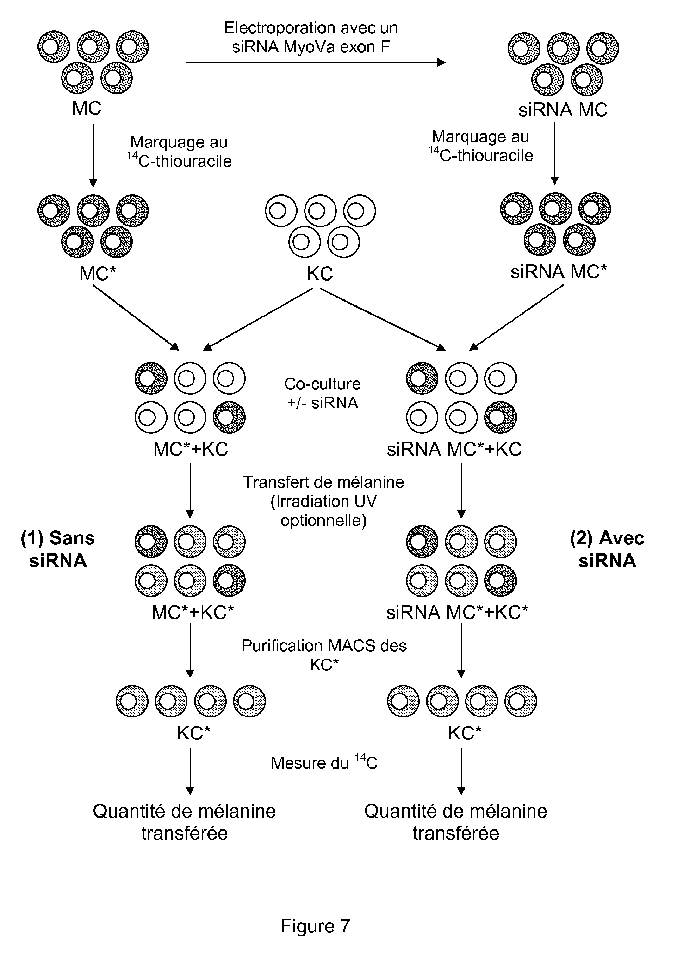

**Figure 7****.** Principe de l'essai d'inhibition in vitro du transfert des mélanosomes des mélanocytes vers les kératinocytes par les siRNA ciblant l'exon F de la protéine myosine Va humaine.

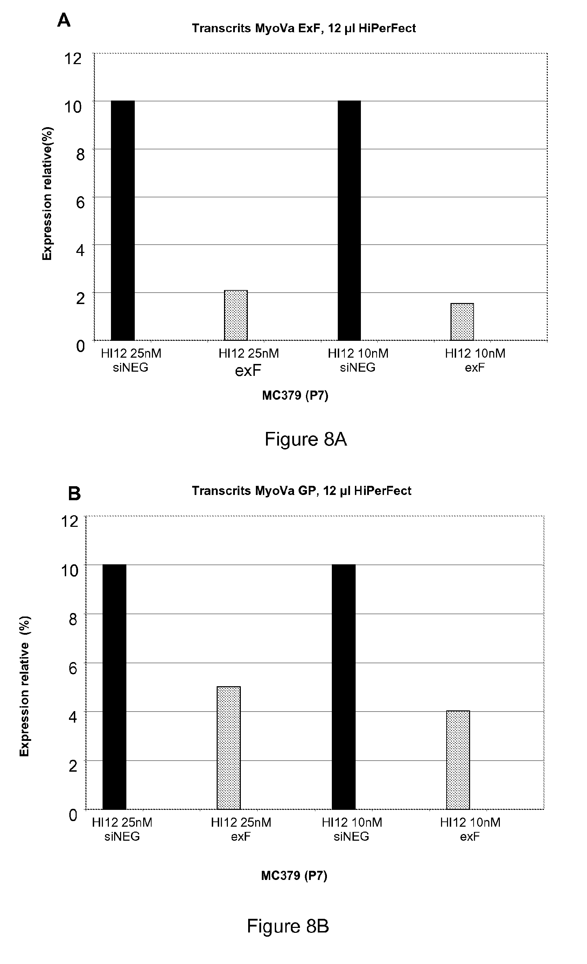

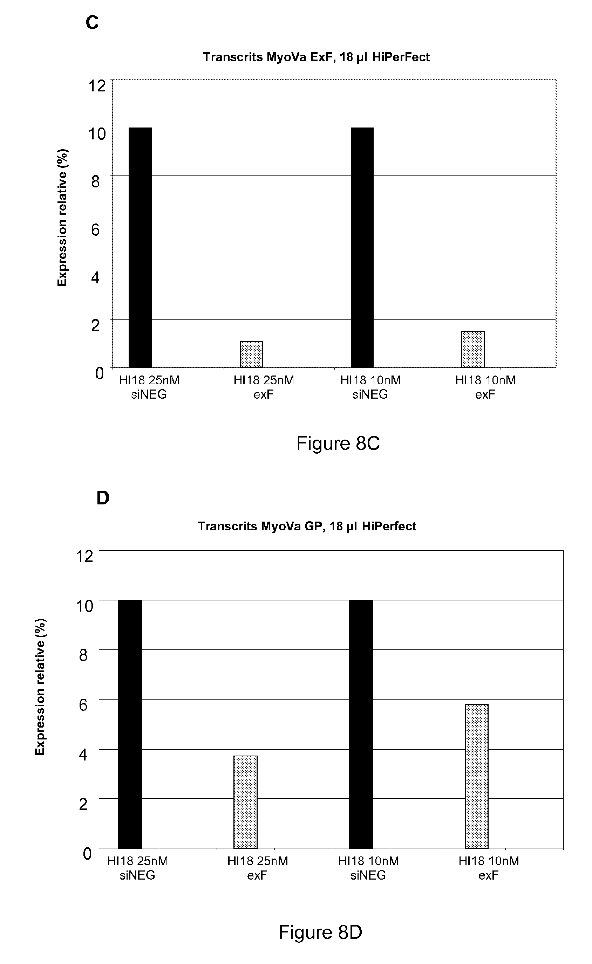

**Figure 8****.** Evaluation de l'inhibition au niveau des transcrits comprenant l'exon F (transcrits MyoVa exF) et des transcrits totaux de la protéine myosine Va détectés au niveaux de la partie GP (transcrits MyoVa GP) dans des MEHP transfectés avec 25nM ou 10nM de siRNA MyoVa n°2 (SEQ ID N°3, exF) ou d'oligonucléotide BLOCK-iT™ Fluorescent oligo (siNEG) et 12 ou 18 µl de réactif HiPerFect. **A.** Transcrits MyoVa exF, 12 µl de réactif HiPerFect **B.** Transcrits MyoVa GP, 12 µl de réactif HiPerFect **C.** Transcrits MyoVa exF, 18 µl de réactif HiPerFect **D.** Transcrits MyoVa GP, 18 µl de réactif HiPerFect.

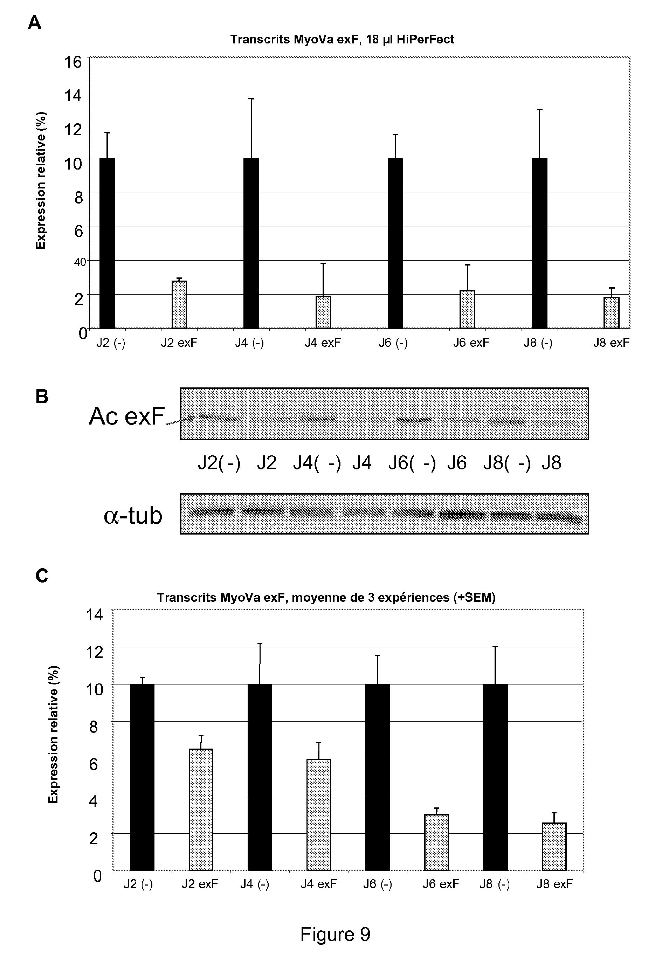

**Figure 9****.** Evaluation de l'inhibition au niveau ARN et au niveau protéique au cours d'une expérience à long terme (J2, J4, J6, J8 après transfection). **A.** Analyse par PCR quantitative en temps réel au niveau ARN **B.** Analyse au niveau protéique par Western blot avec un anticorps dirigé spécifiquement contre les isoformes de la protéine myosine Va contenant l'exon F **C.** Quantification établie avec le logiciel Quantity One software avec normalisation par rapport à la tubuline, à partir des résultats bruts.

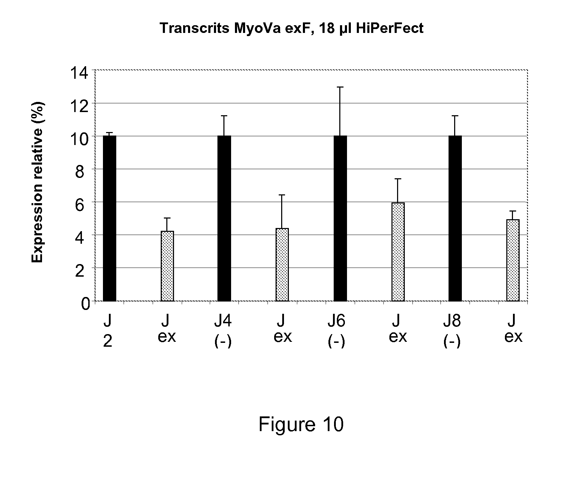

**Figure 10****.** Evaluation de l'inhibition au niveau des transcrits comprenant l'exon F (transcrits MyoVa exF) dans des MEHP transfectés avec le siRNA MyoVa n°1 (SEQ ID N°2, exF) ou l'oligonucléotide BLOCK-iT™ Fluorescent oligo (siNEG) et 18 µl de réactif HiPerFect.

### EXEMPLES

### EXEMPLE 1

### Dessin, synthèse et étude de 3 siRNA ciblant l'exon F de la protéine myosine Va humaine

### 1.1 Dessin et synthèse des 3 siRNA

La séquence du variant d'épissage de la protéine myosine Va humaine comprenant l'exon F (comprend en fait les exons ABCDEF) est représentée sur la Figure 1A.

Au sein de l'exon F, trois siRNA ciblant l'exon F de la protéine myosine Va humaine ont été dessinés et synthétisés en recourant au service de dessin et synthèse de siRNA de la société Eurogentec. Les portions de l'exon F desquelles ces siRNA sont spécifiques sont indiquées sur la Figure 1B.

Les trois siRNA ont été synthétisés avec des fragments 3' en saillie de séquence « TT », comme représenté sur la Figure 1C. Ces trois siRNA sont appelés par la suite MyoVa n°1 (brin sens comprend SEQ ID N°2), MyoVa n°2 (brin sens comprend SEQ ID N°3), ou MyoVa n°3 (brin sens comprend SEQ ID N°4), le numéro de SEQ ID indiqué entre parenthèses correspondant à la séquence du fragment.de l'exon F de la protéine myosine Va humaine comprise dans le brin d'ARN sens du siRNA.

### 1.2 Test de l'efficacité des siRNA après électroporation dans des mélanocytes épidermiques humains primaires (MEHP)

### 1.2.1 Choix du programme d'électroporation

Des mélanocytes épidermiques humains primaires (MEHP) ont été cultivés dans du milieu Ham's F10 (Gibco, Invitrogen Ltd, Paisley, UK) supplémenté avec 2,5% de sérum de veau foetal (SVF), 1% d'Ultroser, 5 ng/ml de facteur de croissance basique des fibroblastes (bFGF), 10 ng/ml d'endothelin-1 (ET-1), 0,33 nM de toxine cholérique (TC), 0,033 mM d'isobutyl-methyl-xanthine (IBMX) et 5,3 nM de 12-O-tetradecanoyl phorbol-13-acetate (TPA). Les cellules ont été cultivées jusqu'à une confluence de 40-70% et utilisées entre les passages 2 et 5.

Dans un premier temps, le plasmide pMAX-GFP (3µg) a été utilisé pour tester trois programmes différents d'électroporation des mélanocytes, à savoir les programmes U16, U20 et U24. 500 000 cellules ont été utilisées par électroporation. Le kit NHEM-Neo Nucleofector (Amaxa, #VDP-1 003) pour mélanocytes humains épidermiques normaux-Neonatal a été utilisé pour toutes les électroporations.

Les cellules électroporées dans des flasques T25 ont été évaluées vivantes à 24-48 heures sous un microscope Arcturus (LCM) équipé d'un filtre fluorescent capable de détecter les signaux de fluorescence verte émis par le plasmide pMAX-GFP.

Les cellules électroporées ont également été cultivées sur des lamelles de verre. 24 heures après électroporation, les cellules ont été fixées dans du méthanol glacé et montées sur des lames de verre avec du fluide de montage (DAKO). L'analyse des cellules concernant l'expression de signaux de fluorescence verte a été faite sous un microscope à fluorescence Zeiss.

Les résultats obtenus sur les cellules vivantes ont montré qu'une quantité égale de cellules mortes est observée quel que soit le programme d'électroporation, mais que la meilleure efficacité d'électroporation est obtenue avec le programme U24. Les résultats obtenus sur les cellules fixées ont confirmé que le programme U24 donne la meilleure efficacité d'électroporation. Le programme U24 a donc été utilisé par la suite dans toutes les autres expériences.

### 1.2.2 Analyse préliminaire de l'efficacité du siRNA MyoVa n°2 (SEQ ID N°3)

500 000 cellules MEHP ont été électroporées avec le plasmide pMAX-GFP (3µg) et les duplexes de siRNA suivants : un contrôle positif ciblant le GAPDH marqué FAM (3 µg ou 2 µM, Ambion), un contrôle négatif BLOCK-iT™ Fluorescent Oligo (Invitrogen), et le duplex de siRNA MyoVa n°2 (SEQ ID N°3) spécifique de l'exon F du gène de la protéine myosine Va. Le programme U24 a été utilisé pour l'électroporation et les cellules ont été récupérées 24 heures après électroporation.

L'analyse a ensuite été réalisée au niveau ARN par RT-PCR quantitative en temps réel (QPCR). Brièvement, après extraction de l'ARN, les échantillons ont été traités à la DNase et l'ADNc a été synthétisé en utilisant la transcriptase inverse iScript (Biorad). Les amorces de QPCR pour l'exon F de la protéine myosine Va (amorce sens : 5' CAGCCTGCAGCACGAGATC 3', SEQ ID NO :5, amorce antisens : 5' TCTTAGGGTCATCTGCATATAATTCCT 3', SEQ ID NO :6) et le GAPDH ont été dessinées en utilisant le logiciel PrimerExpress 2.0 (Applied Biosystems) avec les paramètres Taqman par défaut, en modifiant seulement la contrainte de taille minimum de l'amplicon (75 pb). Les niveaux relatifs d'expression des gènes ont été déterminés à l'aide d'un essai SYBR green | RT-PCR en deux étapes optimisé. La méthode de comparaison des valeurs Ct a été utilisée pour la quantification. Les réactions de PCR ont été réalisées dans un ABI Prism 7000 Sequence Detection System (Applied Biosystem). Pour corriger les différences en quantité d'ARN extrait et en efficacité de synthèse de l'ADNc, les niveaux relatifs d'expression des gènes ont été normalisés par rapport à la moyenne géométrique de trois gènes de ménage (RPL13a, SDHA et UBC) qui ont déjà été utilisés pour des analyses sur des mélanocytes (Vandesompele J et al. Genome Biol.: 3: researchoo34.001 - research0034.001,2002).

Les résultats obtenus montrent que les siRNA utilisés induisent une réduction de l'expression d'ARN des gènes ciblés après électroporation dans des cellules MEHP. En effet, une réduction de 75% pour le GAPDH (Figure 2A) et de 62% pour les transcrits de la protéine myosine Va comprenant l'exon F (Figure 2B) a été observée par rapport au contrôle négatif.

### 1.2.3 Expression des transcrits du gène de la protéine myosine Va dans des cellules MEHP après électroporation avec les siRNA MyoVa n°1 (SEQ ID N°2), Myo Va n°2 (SEQ ID N°3), ou MyoVa n°3 (SEQ ID N°4)

500 000 cellules MEHP ont été électroporées avec le réactif Nucleofector seul (MOCK), le siRNA ciblant GAPDH (1 µM), un siRNA contrôle négatif, ou l'un des trois siRNA MyoVa n°1 (SEQ ID N°2), MyoVa n°2 (SEQ ID N°3), ou MyoVa n°3 (SEQ ID N°4) spécifiques de l'exon F du gène de la protéine myosine Va. Le programme U24 a été utilisé pour l'électroporation et les cellules ont été récupérées 48 heures après électroporation.

L'analyse a ensuite été réalisée au niveau ARN par QPCR comme décrit précédemment sur les transcrits comprenant l'exon F (détection de l'exon F) ou sur l'ensemble des transcrits en détectant la partie globulaire (GP) de la protéine myosine Va. Les amorces de QPCR pour la partie globulaire (GP) de la protéine myosine Va (amorce sens : 5' GCAGTCAATTTGATTCCAGGATT 3', SEQ ID NO :7 ; amorce antisens : 5' TGATCATCATTCAGGTAGTCAGCAT 3', SEQ ID NO :8) ont été dessinées en utilisant le logiciel PrimerExpress 2.0 (Applied Biosystems), tel que décrit précédemment.

Pour les transcrits comprenant l'exon F de la protéine myosine Va, des inhibitions de 85%, 94% et 60% ont été observées pour les siRNA MyoVa n°1 (SEQ ID N°2), MyoVa n°2 (SEQ ID N°3), et MyoVa n°3 (SEQ ID N°4) respectivement (Figure 3A). L'inhibition maximum a donc été obtenue avec le siRNA MyoVa n°2 (SEQ ID N°3).

Concernant l'ensemble des transcrits de la protéine myosine Va, détectés par la partie globulaire (GP), des inhibitions de 39%, 56% et 0% ont été observées pour les siRNA MyoVa n°1 (SEQ ID N°2), MyoVa n°2 (SEQ ID N°3), et MyoVa n°3 (SEQ ID N°4) respectivement (Figure 3B). Cette inhibition réduite peut notamment s'expliquer par le fait que les siRNA utilisés ciblent spécifiquement l'exon F et n'induisent donc pas la dégradation des transcrits ne comprenant pas l'exon F.

### 1.2.4 Conclusions

Ces premiers résultats montrent clairement qu'il est possible de réduire spécifiquement et significativement la quantité de transcrits de la protéine myosine Va humaine comprenant l'exon F en utilisant les trois siRNA synthétisés, sans affecter pour autant les transcrits ne comprenant pas l'exon F.

Les résultats les plus significatifs ont été obtenus avec le siRNA MyoVa n°2 (SEQ ID N°3).

### 1.3 Optimisation de la concentration de siRNA

### 1.3.1 Détermination d'une concentration minimale à efficacité maximale

Dans le but de déterminer la concentration minimale en siRNA permettant une efficacité maximale, différentes concentrations allant de 0,05 à 2 µM ont été testées par le même protocole que précédemment décrit. Le siRNA contrôle négatif et le siRNA ciblant le GAPDH ont été utilisés à une concentration de 1 µM.

Le programme U24 a été utilisé et les cellules ont été analysées à 48 heures après électroporation.

L'analyse a été réalisée par QPCR comme précédemment décrit sur les transcrits comprenant l'exon F (détection spécifique de l'exon F).

Pour le siRNA MyoVa n°1 (SEQ ID N°2), une réduction en transcrits comprenant l'exon F de 47,5%, 31%, 73%, 73%, et 79% a été observée par rapport au contrôle négatif pour les concentrations de 0,05 ; 0,1 ; 0,25 ; 0,5 et 1 µM respectivement (Figure 4A). La meilleure efficacité est donc obtenue avec une concentration de 1 µM, mais une inhibition presque aussi efficace est obtenue avec les concentrations de 0,25 et 0,5 µM.

En ce qui concerne le siRNA MyoVa n°2 (SEQ ID N°3), une forte réduction a été observée à toutes les concentrations testées (Figure 4B). L'efficacité maximum a été observée pour une concentration de 1 µM, mais l'inhibition obtenue avec une concentration de 0,5 µM est presque aussi forte.

Une concentration optimale en siRNA se situe donc autour de 0,5-1 µM.

### 1.3.2 Influence de la concentration en siRNA sur la viabilité des cellules

L'influence de la concentration en siRNA sur la viabilité des cellules a ensuite été analysée avec le siRNA MyoVa n°2 (SEQ ID N°3). Pour ce faire, la même gamme de concentrations (0,05 à 2 µM) a été testée en parallèle avec un test MTS (Promega Benelux) qui permet de déterminer le nombre de cellules viables. Les cellules ont été analysées à 48 heures après électroporation par le programme U24.

Les résultats sont représentés sur la Figure 5 et montrent que la viabilité des cellules n'est significativement réduite (environ 20%) que pour des concentrations en siRNA de 1 ou 2 µM. En dessous de 1 µM, la viabilité des cellules est supérieure à 90%.

### 1.3.3 Conclusions

Ces résultats montrent que l'effet des siRNA ciblant l'exon F de la protéine myosine Va est dose-dépendant, et qu'une efficacité maximale peut être obtenue pour une concentration comprise entre 0,5 et 1µM.

De plus, en dessous de 1 µM, la présence des siRNA n'affecte pas la viabilité des cellules MEHP.

Une concentration de 0,5 µM semble donc optimale, puisqu'elle combine une forte efficacité et une forte viabilité cellulaire. Cette concentration a été utilisée dans toutes les expériences suivantes décrites ci-dessous.

### 1.4 Analyse au cours du temps

Pour analyser l'effet dans le temps des siRNA selon l'invention, des cellules MEHP électroporées avec 0,5 µM de siRNA MyoVa n°1 (SEQ ID N°2) ou MyoVa n°2 (SEQ ID N°3) ont été analysées par QPCR comme précédemment décrit à 24, 48, 72 et 96 heures après électroporation. Les cellules traitées avec les contrôles positif ou négatif (0,5 µM) ont été analysées à 48 heures après électroporation.

Pour le siRNA MyoVa n°1 (SEQ ID N°2), la réduction en transcrits comprenant l'exon F est maximale à 24h (plus de 90%) et diminue ensuite à 48h (65%) et 72 h (60%), le niveau d'expression retournant à la normale à 96 h (voir Figure 6A).

En ce qui concerne le siRNA MyoVa n°2 (SEQ ID N°3), une réduction de plus de 85% a été observée à 24 et 48 heures, qui diminue ensuite à 72 et 96 heures, tout en restant significative par rapport au contrôle négatif (Figure 6B).

Ainsi, ces résultats montrent que l'efficacité maximale est obtenue à 24-48 heures après électroporation, et que l'efficacité du siRNA MyoVa n°2 (SEQ ID N°3) est supérieure dans le temps à celle du siRNA MyoVa n°1 (SEQ ID N°2).

### 1.5 Conclusions

Ainsi, les résultats présentés ci-dessus montrent clairement qu'il est possible de réduire très fortement la quantité de transcrits de la protéine myosine Va comprenant l'exon F en utilisant des siRNA ciblant ledit exon F. Trois siRNA différents ont été testés, et chacun de ces siRNA permet de réduire la quantité de transcrits de la protéine myosine Va comprenant l'exon F. Le siRNA MyoVa n°1 (SEQ ID N°2), et surtout le siRNA MyoVa n°2 (SEQ ID N°3), sont particulièrement efficaces.

De plus, l'effet de ces siRNA ciblant l'exon F de la protéine myosine Va est dose-dépendant et peut être obtenu avec une efficacité maximale à une concentration n'affectant pas la viabilité des cellules.

L'efficacité varie également au cours du temps et est maximale à 24-48 heures après électroporation.

### EXEMPLE 2

### Influence de la présence d'un siRNA ciblant l'exon F de la protéine myosine Va sur le transfert des mélanosomes des mélanocytes vers les kératinocytes in vitro

### 2.1 Principe de l'essai

Afin de tester l'influence des siRNA selon l'invention ciblant l'exon F de la protéine myosine Va sur le transfert de la mélanine des mélanocytes vers les kératinocytes, un essai in vitro a été mis au point.

Le principe de cet essai est représenté sur la Figure 7.

Des mélanocytes (MC) humains normaux sont ou non électroporés avec un siRNA ciblant l'exon F de la protéine myosine Va aux concentrations de 0,1 µM ; 0,25 µM ; et 0,5 µM. Ensuite, les mélanocytes électroporés (siRNA MC) ou non (MC) sont cultivés dans un milieu TFA enrichi en présence de 2[2-¹⁴C]thiouracile, qui est incorporé dans la mélanine produite par les MC, ce qui permet d'obtenir des mélanocytes contenant de la mélanine marquée au ¹⁴C (MC* ou siRNA MC*).

Les MC* ou siRNA MC* sont mis en culture dans un milieu pour kératinocytes sans sérum et avec une faible teneur en calcium (K-SFM, Gibco) avec des kératinocytes (KC) ayant subit 1 ou 2 passages dans ce même milieu. Les MC* ou siRNA MC* et les KC sont mis en culture dans un ratio de 1 pour 3.

La co-culture est ensuite optionnellement irradiée aux UV pour stimuler le transfert des mélanosomes des MC* ou siRNA MC* vers les KC. En effet, il est connu que l'irradiation par les UV stimule le transfert des mélanosomes des mélanocytes vers les kératinocytes. Après co-culture et irradiation UV éventuelle, les kératinocytes sont radioactifs au ¹⁴C (KC*) proportionnellement à la quantité de mélanine transférée à partir des MC* ou siRNA MC*.

Ensuite, les KC* sont purifiés par sélection négative en utilisant la technologie MACS (Miltenyi Biotech), à l'aide d'un anticorps anti-CD117 PE qui se fixe sur les mélanocytes et de billes anti-PE.

Enfin, la radioactivité ¹⁴C des KC* purifiés est mesurée.

### 2.2 Résultats

Les résultats obtenus montrent qu'en absence d'électroporation avec un siRNA ciblant l'exon F de la protéine myosine Va, l'irradiation UV permet de stimuler efficacement le transfert de la mélanine des mélanocytes vers les kératinocytes.

En revanche, en cas d'électroporation avec un siRNA ciblant l'exon F de la protéine myosine Va, la quantité de mélanine radioactive au ¹⁴C transférée aux kératinocytes après irradiation UV est réduite par rapport au contrôle sans électroporation.

### 2.3 Conclusions

Ces résultats montrent clairement que l'utilisation de siRNA ciblant l'exon F de la protéine myosine Va permet de réduire le transfert de la mélanine des mélanocytes vers les kératinocytes, validant ainsi la stratégie utilisée par les inventeurs pour réduire la pigmentation de la peau.

### EXEMPLE 3

### Efficacité des siRNA ciblant l'exon F de la protéine myosine Va après transfection dans des mélanocytes épidermiques humains primaires (MEHP)

### 3.1 Transfection de MEHP avec des siRNA ciblant l'exon F de la protéine myosine Va

### 3.1.1 Optimisation de la transfection avec les réactifs de transfection HiPerFect

200 000 MEHP (dans 0,5 ml de milieu/plaque de 6 puits) ont été transfectés avec le siRNA MyoVa n°2 (SEQ ID N°3) et l'oligonucléotide BLOCK-iT™ Fluorescent oligo (Invitrogen) comme contrôle négatif. Pour chaque transfection, 25nM ou 10nM de chaque siRNA a été utilisé en combinaison avec soit 12 soit 18 µl de réactif HiPerFect (Qiagen).

L'évaluation de l'inhibition au niveau ARN, soit uniquement pour les transcrits comprenant l'exon F (transcrits MyoVa exF), soit pour l'ensemble des transcrits de la protéine myosine Va détectés au niveaux de la partie GP (transcrits MyoVa GP), a été mesurée par PCR quantitative en temps réel comme décrit précédemment au paragraphe Exemple 1.2.2.

Les résultats obtenus avec 12 µl ou 18 µl de réactif HiPerFect sur les transcrits MyoVa exF ou MyoVa GP sont présentés sur la Figure 8, et montrent que :
- pour les transcrits MyoVa exF avec 12 µl de réactif HiPerFect, une inhibition de 80% et 85% est observée pour 25nM et 10nM de siRNA MyoVa n°2 (SEQ ID N°3) respectivement (Figure 8A).
- pour les transcrits MyoVa GP avec 12 µl de réactif HiPerFect, une inhibition de 50% et 60% est observée pour 25nM et 10nM de siRNA MyoVa n°2 (SEQ ID N°3) respectivement (Figure 8B).
- pour les transcrits MyoVa exF avec 18 µl de réactif HiPerFect, une inhibition de 90% et 85% est observée pour 25nM et 10nM de siRNA MyoVa n°2 (SEQ ID N°3) respectivement (Figure 8C).
- pour les transcrits MyoVa GP avec 18 µl de réactif HiPerFect, une inhibition de 63% et 58% est observée pour 25nM et 10nM de siRNA MyoVa n°2 (SEQ ID N°3) respectivement (Figure 8D).

### 3.1.2 Conclusion

Ces résultats montrent une inhibition significative et spécifique du nombre de transcrits de protéine myosine Va comprenant l'exon F (transcrits Myo Va exF) par transfection de MEHP avec le siRNA MyoVa n°2 (SEQ ID N°3) et le réactif HiPerFect. L'inhibition la plus efficace est obtenue avec 25 nM 25nM de siRNA n°2 (SEQ ID N°3), et 18 µl de réactif HiPerFect.

Ces conditions optimales sont utilisées dans toutes les expériences de l'Exemple 3 décrites ci-dessous.

### 3.2 Evaluation de l'inhibition obtenue après transfection de MEHP avec le siRNA MyoVa n°2 (SEQ ID N°3)

### 3.2.1 Evaluation de l'inhibition au niveau ARN et au niveau protéique au cours d'une expérience à long terme

800 000 MEHP (dans 2,5 ml de milieu/plaque P60) ont été transfectés avec 18 µl de réactif HiPerFect et 25nM de siRNA MyoVa n°2 (SEQ ID N°3) ou avec 25nM d'oligonucléotide BLOCK-iT™ Fluorescent oligo (contrôle négatif). L'effet de l'inhibition a été évalué sur une période de 8 jours. 1/3 des cellules ont été utilisées pour la purification d'ARN et 2/3 des cellules ont été utilisées pour purifier les lysats cellulaires.

L'évaluation de l'inhibition au niveau ARN pour les transcrits comprenant l'exon F (transcrits MyoVa exF) a été mesurée par PCR quantitative en temps réel comme décrit précédemment au paragraphe Exemple 1.2.2.

Pour corréler l'inhibition observée au niveau ARN avec l'expression au niveau protéique, un anticorps polyclonal dirigé spécifiquement contre les isoformes de la protéine myosine Va contenant l'exon F a été développé par la société Eurogentec. La réactivité et la spécificité de l'anticorps purifié a été testé par analyse par Western blot et par immunohistochimie.

Ensuite, les lysats cellulaires obtenus au cours de l'expérience ont été analysés par Western blot.

### Au niveau ARN

Les résultats sont présentés sur la Figure 9A et montrent que, comparé aux contrôles négatifs, une réduction de 72%, 81%, 78% et 82% a été observée pour les transcrits MyoVa exF aux différents points dans le temps (J2, J4, J6 et J8 respectivement).

### Au niveau protéique

Les résultats de l'analyse des lysats cellulaires par Western blot sont présentés sur les Figure 9B et C représentant respectivement les résultats bruts du Western blot et la quantification obtenue avec le logiciel Quantity One software (Biorad) avec normalisation par rapport à la tubuline.

Ces résultats montrent que, par rapport au contrôles négatifs, une réduction de 35%, 40%, 70% et 75% a été observée pour les transcrits MyoVa exF aux différents points dans le temps (J2, J4, J6 et J8 respectivement).

### 3.2.2 Effet de l'inhibition induite par le siRNA MyoVa exF n°2 (SEQ ID N°3) sur la localisation/distribution des mélanosomes dans les MEHP

Il a été précédemment montré que les transcrits MyoVa exF sont impliqués dans la capture des mélanosomes dans le réseau d'actine subcortical. L'inhibition des transcrits MyoVa exF pourrait donc résulter dans une capture diminuée des mélanosomes à la périphérie des dendrites des mélanocytes, et éventuellement dans une accumulation périnucléaires des mélanosomes.

Pour tester cette hypothèse, des transfections avec 25nM de siRNA MyoVa exF n°2 (SEQ ID N°3) et 25nM d'un siRNA contrôle négatif ont été réalisées sur 300 000 MEHP ensemencés sur des lamelles de verre rondes. Les MEHP ont été fixés dans du paraformaldehyde 3% à différents points dans le temps après transfection (jour 3, jour 6 et jour 8).

Ensuite, pour les expériences d'immunohistochimie, l'anticorps spécifique de l'exon F de la protéine myosine Va (dilution 1/500) (voir ci-dessus) a été utilisé en combinaison avec un anticorps monoclonal NKI-beteb (dilution 1/40) dirigé contre la proteine (pré)-mélanosomale *silver* (Sanbio B.V., Uden, The Netherlands). L'analyse a alors été réalisée par microscopie confocale.

### Résultats :

### Jour 3 après transfection

Aucune différence n'a été observée dans la distribution des mélanosomes (présents à la périphérie et aux extrémités des dendrites) entre les MEHP transfectés avec le siRNA contrôle négatif et les MEHP transfectés avec le siRNA MyoVa exF n°2 (SEQ ID N°3).

### Jour 6 après transfection

Le co-marquage avec l'anticorps anti NKI-beteb et l'anticorps anti exon F de la protéine myosine Va montre un changement dans la distribution des melanosomes dans les MEHP transfectés avec le siRNA MyoVa exF n°2 (SEQ ID N°3) : les mélanosomes sont situés principalement dans la zone périnucléaire. En outre, le marquage pour les isoformes de la protéine myosine Va comprenant l'exon F est absent ou faible au niveau des extrémités des dendrites.

### Jour 8 après transfection

Les mêmes résultats que ceux observés à jour 6 sont observés.

### Conclusion

L'analyse immunohistochimique de MEHP transfectés par le siRNA MyoVa exF n°2 (SEQ ID N°3) ou par un siRNA contrôle avec l'anticorps anti NKI-beteb et l'anticorps anti exon F de la protéine myosine Va permet de visualiser le transport intracellulaire des mélanosomes (par NKI-beteb) et également de détecter l'expression des isoformes de la protéine myosine Va contenant l'exon F.

Les résultats obtenus permettent de conclure que l'inhibition des isoformes de la protéine myosine Va contenant l'exon F conduit à une distribution anormale des mélanosomes dans les MEHP, c'est-à-dire une distribution périnucléaire au lieu d'une localisation périphérique avec présence au niveau des extrémités des dendrites dans les contrôles négatifs, à jours 6 et 8 après transfection.

### 3.3 Confirmation de l'effet d'inhibition des transcrits MyoVa exF après transfection de MEHP avec le siRNA MyoVa n°1 (SEQ ID N°2)

Dans ces expériences, un siRNA différent, à savoir le siRNA MyoVa n°1 (SEQ ID N°2) a été utilisé pour la transfection des MEHP de façon à confirmer que l'inhibition est bien spécifique de l'exon F de la protéine myosine Va et à exclure un effet non spécifique de type artéfactuel.

### 3.3.1 Evaluation de l'inhibition au niveau ARN et au niveau protéique au cours d'une expérience à long terme

La même expérience qu'au paragraphe 3.2.1 a été reproduite avec le siRNA MyoVa n°1 (SEQ ID N°2) au lieu du siRNA MyoVa n°2 (SEQ ID N°3).

Les résultats au niveau ARN sont présentés sur la Figure 10 et montrent une réduction de 58%, 59%, 41% et 51% dans les aux différents points dans le temps (J2, J4, J6 et J8 respectivement).

En outre, les résultats obtenus au niveau protéique étaient en corrélation avec ceux obtenus au niveau ARN (données non montrées).

### 3.3.2 Effet de l'inhibition induite par le siRNA MyoVa exF n°1 (SEQ ID N°2) sur la localisation/distribution des mélanosomes dans les MEHP

La même expérience qu'au paragraphe 3.2.2 a été reproduite avec le siRNA MyoVa n°1 (SEQ ID N°2) au lieu du siRNA MyoVa n°2 (SEQ ID N°3).

Les résultats obtenus à jour 8 après transfection avec le siRNA MyoVa n°1 (SEQ ID N°2) montrent comme pour le siRNA MyoVa n°2 (SEQ ID N°3) une distribution périnucléaire des mélanosomes dans les MEHP par comparaison aux contrôles négatifs (données non montrées).

### 3.3.3 Conclusions

Bien que l'inhibition des isoformes de la protéine myosine Va comprenant l'exon F (transcrits MyoVa exF) soit moins efficace avec le siRNA MyoVa exF n°1 (SEQ ID N°2) qu'avec le siRNA MyoVa exF n°2 (SEQ ID N°3), l'expression des transcrits MyoVa exF reste significativement réduite en utilisant le siRNA MyoVa exF n°1 (SEQ ID N°2), aussi bien au niveau ARN qu'au niveau protéique.

En outre, l'analyse immunohistochimique sur des MEHP transfectés fixés révèle également une localisation et un transport perturbés des mélanosomes dans les MEHP. On peut donc conclure que l'inhibition des isoformes de la protéine myosine Va comprenant l'exon F et l'effet phénotypique observé induits par l'utilisation des siRNA MyoVa exF n°1 et 2 (SEQ ID N° 2 et 3) sont spécifiques et non artéfactuels.

### EXEMPLE 4

### Induction d'un effet phénotypique par inhibition à long terme des transcrits de la protéine myosine Va comprenant l'exon F (transcrits MyoVa exF)

De façon à obtenir dans des MEHP une inhibition stable à long terme des isoformes de la protéine myosine Va comprenant l'exon F, des vecteurs lentiviraux exprimant de petits ARN en épingle (short hairpin RNA, ou shRNA) dirigés contre l'exon F de la protéine myosine Va ont été construits.

### 4.1 Matériels et méthodes

Les séquences des shRNA ont été développées à partir de la séquence siRNA du siRNA le plus efficace pour inhiber les transcrits MyoVa exF, c'est-à-dire le siRNA MyoVa n°2 (comprenant la séquence ARN de la séquence génique SEQ ID N°3). Plus précisément, un oligonucléotide double brin constitué des séquences suivantes a été synthétisé :
- brin sens : 5'-GATC**GTATCAATCATATCGGATT**CTCAAGAGA**AATCCGATATG ATTGATAC***TTTTT*-3'(SEQ ID N°15)
- brin antisens : 5'-AGCT*AAAAA***GTATCAATCATATCGGATT**TCTCTTGAG**AATCCGAT ATGATTGATAC**-3' (SEQ ID N°16),
   où
- les portions en gras correspondent aux deux portions complémentaires du shRNA appariées par des liaisons de type Watson-Crick, correspondant à la séquence ARN de la séquence génique SEQ ID N°3 du brin sens du siRNA MyoVa n°2 et sa séquence complémentaire,
- la portion de séquence non modifiée correspond à la séquence de la structure de type épingle à cheveux liant les deux brins complémentaires,
- la portion soulignée correspond à un fragment 5' en saillie permettant de cloner l'oligonucléotide double brin dans un vecteur d'expression, et
- la portion en italique correspond à une séquence de terminaison de la transcription initiée par un promoteur RNApolIII type H1.

Cet oligonucléotide double brin a alors été cloné dans un vecteur lentiviral sous contrôle d'un promoteur RNApolIII type H1.

Cela permet de produire dans les cellules transfectées des shRNA de séquence :
5'-**GUAUCAAUCAUAUCGGAUUC**UCAAGAGA**AAUCCGAUAUGAUUGAUAC**-3' (SEQ ID N°17), où les portions en gras correspondent aux deux portions complémentaires appariées par des liaisons de type Watson-Crick (correspondant à la séquence ARN de la séquence génique SEQ ID N°3 du brin sens du siRNA MyoVa n°2 et sa séquence complémentaire), et la portion sans modification correspond à la séquence de la structure de type épingle à cheveux liant les deux brins complémentaires.

Des vecteurs lentiviraux exprimant des shRNAs brouillés ont également été générés pour être utilisés comme contrôle négatif. Dans ce cas, les séquences brins sens et antisens de l'oligonucléotide cloné dans le vecteur lentiviral sous contrôle d'un promoteur RNApolIII type H1 étaient les séquences suivantes :
- brin sens : 5'-GATC**ATTATCTAGGAGATATCACC**TCAAGAGA**GTGATATCTCC TAGATAA***TTTTT*-3' (SEQ ID N°18)
- brin antisens : 5'-AGCT*AAAAA***ATTATCTAGGAGATATCAC**TCTCTTGAG**GTGATAT CTCCTAGATAAT**-3' (SEQ ID N°19)
   où
- les portions en gras correspondent aux deux portions complémentaires du shRNA appariées par des liaisons de type Watson-Crick, correspondant aux bases dans le désordre de la séquence ARN de la séquence génique SEQ ID N°3 du brin sens du siRNA MyoVa n°2 et sa séquence complémentaire,
- la portion de séquence non modifiée correspond à la séquence de la structure de type épingle à cheveux liant les deux brins complémentaires,
- la portion soulignée correspond à un fragment 5' en saillie permettant de cloner l'oligonucléotide double brin dans un vecteur d'expression, et
- la portion en italique correspond à une séquence de terminaison de la transcription initiée par un promoteur RNApolIII type H1.

Cela permet de produire dans les cellules transfectées des shRNA de séquence : 5'-**AUUAUCUAGGAGAUAUCAC**CUCAAGA**GAGUGAUAUCUCCUAGAUAA-**3' (SEQ ID N°20), où les portions en gras correspondent aux deux portions complémentaires appariées par des liaisons de type Watson-Crick (correspondant aux bases dans le désordre de la séquence ARN de la séquence génique SEQ ID N°3 du brin sens du siRNA MyoVa n°2 et sa séquence complémentaire), et la portion sans modification correspond à la séquence de la structure de type épingle à cheveux liant les deux brins complémentaires.

### 4.2 Efficacité de transduction

L'efficacité de transduction dans des MEHP a été déterminée par analyse par FACS sur la base de la présence d'un marqueur GFP dans lesvecteurs lentiviraux mentionnés ci-dessus.

La transduction de 100 000 MEHP avec des lentivirus à une multiplicité d'infection (MI) de 10 conduit à une efficacité de transduction de 90% après deux séries d'infection.

### 4.3 Evaluation de l'inhibition au niveau ARN et au niveau protéique

Pour évaluer l'effet d'inhibition au niveau ARN et au niveau protéique, 600 000 MEHP ont été transduits à une MI de 10 avec des vecteurs lentiviraux contenant des shRNAs MyoVa exF n°2 ou des shRNAs brouillés (contrôle négatif), respectivement.

Les résultats montrent, aussi bien au niveau ARN qu'au niveau protéique, une forte réduction des isoformes de la protéine myosine Va comprenant l'exon F dans les cellules transduites avec les vecteurs lentiviraux contenant des shRNAs MyoVa exF n°2 par rapport au contrôle négatif.

### 4.4 Effet de l'inhibition sur le transport des mélanosomes

Ensuite une analyse immunohistochimique de MEHP transduits avec des vecteurs lentiviraux contenant des shRNAs MyoVa exF n°2 ou des shRNAs brouillés (contrôle négatif), a été réalisée. Les résultats montrent une distribution périnucléaire des mélanosomes dans les MEHP transduits avec les vecteurs lentiviraux contenant des shRNAs MyoVa exF n°2 par rapport au contrôle négatif.

Ces résultats confirment les effets précédemment observés en cas d'inhibition des transcrits MyoVa exF par utilisation de siRNAs synthétiques

### 4.5 Effet de l'inhibition des transcrits MyoVa exF sur le transfert de la mélanine des mélanocytes aux kératinocytes dans un modèle 3D d'épiderme reconstruit.

50 000 MEHP ont été transduits avec les vecteurs lentiviraux exprimant les shRNAs MyoVa exF n°2. Ces MEHP transduits de façon stable ont été introduits, avec 500 000 kératinocytes, dans un modèle de peau reconstruite. Des MEHP transduits de façon stable avec des vecteurs lentiviraux exprimant des shRNAs brouillés ont été utilisés comme contrôles négatifs. Les peaux reconstruites ont ensuite été irradiées ou non avec des UV simulant le soleil.

L'inspection visuelle des peaux reconstruites montre alors qu'aucune augmentation de la pigmentation n'est observée après irradiation au niveau des peaux contenant des MEHP transduits avec les vecteurs lentiviraux exprimant les shRNAs MyoVa exF n°2, contrairement aux peaux contenant des MEHP transduits avec les vecteurs lentiviraux exprimant les shRNAs brouillés. Une évaluation quantitative de la pigmentation par un marquage Fontana-Masson et analyse d'image colorimétrique digitale a ensuite permis de confirmer ces observations.

La distribution des mélanosomes dans les mélanocytes et les kératinocytes a ensuite été examinée par microscopie électronique. Ces analyses ont montré une stimulation du transfert de pigments des mélanocytes vers les kératinocytes dans les peaux reconstruites irradiées aux UV contenant des MEHP transduits avec les vecteurs lentiviraux exprimant les shRNAs brouillés, par rapport aux mêmes peaux non irradiées. Au contraire, dans les peaux reconstruites irradiées aux UV contenant des MEHP transduits avec les vecteurs lentiviraux exprimant des shRNA MyoVa exF n°2, seul un transfert réduit de mélanine a été observé par rapport aux peaux contrôles non irradiées.

### 4.6 Conclusions

Ainsi, ces résultats démontrent qu'une transfection stable de MEHP avec des vecteurs lentiviraux exprimant un shRNA ciblant spécifiquement l'exon F de la protéine myosine Va humaine permet d'interférer de façon significative avec la quantité de transcrits MyoVa exF et d'isoformes de la protéine myosine Va comprenant l'exon F, ainsi qu'avec le transport des mélanosomes et leur transfert des mélanocytes vers les kératinocytes.

## Revendications

1. siRNA isolé comprenant un brin d'ARN sens et un brin d'ARN antisens complémentaire formant ensemble un duplex d'ARN, **caractérisé en ce que** le brin d'ARN sens comprend une séquence qui possède au plus un nucléotide distinct par rapport à un fragment de 14 à 30 nucléotides contigus de la séquence nucléotidique de l'exon F du gène codant pour la protéine myosine Va.

2. siRNA selon la revendication 1, **caractérisé en ce que** le brin d'ARN sens comprend une séquence identique à un fragment de 14 à 30 nucléotides contigus de la séquence nucléotidique de l'exon F du gène codant pour la protéine myosine Va.

3. siRNA selon les revendications 1 ou 2, **caractérisé en ce que** la séquence nucléotidique de l'exon F du gène codant pour la protéine myosine Va est la séquence humaine SEQ ID N°1.

4. siRNA selon la revendication 3, **caractérisé en ce que** ledit fragment de 14 à 30 nucléotides contigus de la séquence nucléotidique de l'exon F du gène codant pour la protéine myosine Va humaine est constitué d'une séquence nucléotidique choisie parmi SEQ ID N°2, SEQ ID N°3, et SEQ ID N°4.

5. siRNA selon la revendication 4, **caractérisé en ce que** ledit fragment de 14 à 30 nucléotides contigus de la séquence nucléotidique de l'exon F du gène codant pour la protéine myosine Va est constitué de la séquence nucléotidique SEQ ID N°3.

6. siRNA selon la revendication 5, **caractérisé en ce que** ledit siRNA est un shRNA constitué d'une seule molécule d'ARN simple brin dans laquelle deux portions complémentaires sont appariées par des liaisons de type Watson-Crick et sont liées d'un côté de façon covalente par une structure de type épingle à cheveux, où ledit shRNA comprend ou est constitué de la séquence SEQ ID N°17.

7. siRNA selon l'une quelconque des revendications 1 à 5, **caractérisé en ce que** les brins d'ARN sens et/ou antisens comprennent en outre un fragment 3' en saillie de 2 à 4 nucléotides naturels ou modifiés de type LNA.

8. siRNA selon l'une quelconque des revendications 1 à 7, **caractérisé en ce que** le brin d'ARN sens et/ou le brin d'ARN antisens comprennent au moins une modification chimique au niveau de leurs parties sucres, de leurs parties nucléobases ou de leur squelette internucléotidique.

9. siRNA selon l'une quelconque des revendications 1 à 8, en tant qu'agent cosmétique.

10. siRNA selon l'une quelconque des revendications 1 à 8, en tant que médicament.

11. Composition comprenant au moins un siRNA selon l'une quelconque des revendications 1 à 8 et un support acceptable.

12. Composition selon la revendication 11, **caractérisée en ce qu'**elle est administrée par voie topique.

13. Composition selon les revendications 11 ou 12, **caractérisée en ce qu'**elle comprend en outre au moins un autre agent dépigmentant.

14. Utilisation d'au moins un siRNA selon l'une quelconque des revendications 1 à 8 ou d'une composition selon l'une quelconque des revendications 11 à 13 comme agent cosmétique pour la dépigmentation ou le blanchiment de la peau.

15. Utilisation d'au moins un siRNA selon l'une quelconque des revendications 1 à 8 ou d'une composition selon l'une quelconque des revendications 11 à 13 pour la fabrication d'un médicament destiné au traitement ou à la prévention de l'hyperpigmentation de la peau.
